# EUROPEAN PATENT SPECIFICATION

(11) **EP 3 080 146 B1**
(45) Date of publication and mention of the grant of the patent: **27.03.2019**
(21) Application number: 14731957.8
(22) Date of filing: 20.06.2014
(51) Int. Cl.: A01H 5/02, A01H 6/32, C12N 15/82, C07K 14/415, C12N 9/10

(54) **GENE ENCODING A MUTANT PROTEIN PROVIDING A DECORATIVE FLOWERING PHENOTYPE IN PLANTS**
FÜR EIN MUTANTES PROTEIN FÜR EINEN DEKORATIVEN BLÜTENPHÄNOTYP BEI PFLANZEN CODIERENDES GEN
GÈNE CODANT POUR UNE PROTÉINE MUTANTE INTRODUISANT UN PHÉNOTYPE DE FLORAISON DÉCORATIVE CHEZ LES PLANTES

(30) Priority: 12.12.2013 WO PCT/EP2013/076332
(43) Date of publication of application: 19.10.2016
(73) Proprietor: Dümmen Group B.V., 2678 PS De Lier (NL)
(72) Inventor: VLIELANDER, Izaak, Johannes, 2678 PA De Lier (NL); LANG, Chunting, 2678 PS De Lier (NL); MARIS, Paulus, Cornelis, 2678 PS De Lier (NL); PEETERS, Roger, Adrianus, 6003 CH Weert (NL)
(74) Representative: van Kooij, Adriaan
(86) International application number: PCT/EP2014/063019
(87) International publication number: WO 2015/086165

(56) References cited:
- US-P2- 18 011
- YOSHIHIRO HASE ET AL: "FRL1 is required for petal and sepal development in Arabidopsis", THE PLANT JOURNAL, vol. 24, no. 1, 1 October 2000 (2000-10-01), pages 21-32, XP055135828, ISSN: 0960-7412, DOI: 10.1046/j.1365-313x.2000.00851.x
- VÉRONIQUE BERGOUGNOUX ET AL: "Both the adaxial and abaxial epidermal layers of the rose petal emit volatile scent compounds", PLANTA ; AN INTERNATIONAL JOURNAL OF PLANT BIOLOGY, SPRINGER, BERLIN, DE, vol. 226, no. 4, 23 May 2007 (2007-05-23), pages 853-866, XP019542330, ISSN: 1432-2048, DOI: 10.1007/S00425-007-0531-1

## Description

The present invention relates to proteins capable of providing a decorative flowering phenotype to plants and especially to plants belonging to the *Kalanchoe* genus. The present invention also relates to nucleic acid sequences, or cDNA sequences, and genes encoding the present proteins. The present invention further relates to use of the present proteins, nucleic acid sequences and genes for selecting decorative flowering *Kalanchoe* plants.

*Kalanchoe* is a genus of about 125 species of tropical, succulent flowering plants in the family *Crassulaceae.* Only one species of this genus is known to originate from the Americas, 56 from southern & eastern Africa and 60 species from Madagascar. *Kalanchoe* plants are also found in south-eastern Asia and China.

Known species belonging to the *Kalanchoe* genus are *Kalanchoe adelae*; *Kalanchoe arborescens, Kalanchoe beauverdii, Kalanchoe beharensis, Kalanchoe bentii, Kalanchoe blossfeldiana, Kalanchoe bouvetii, Kalanchoe bracteata, Kalanchoe campanulata, Kalanchoe crenata*, *Kalanchoe crundallii*, *Kalanchoe daigremontiana*, *Kalanchoe delagoensis, Kalanchoe dinklagei, Kalanchoe eriophylla, Kalanchoe farinacea, Kalanchoe fedtschenkoi*, *Kalanchoe figuereidoi, Kalanchoe flammea, Kalanchoe gastonis, Kalanchoe glaucescens, Kalanchoe gracilipes, Kalanchoe grandidieri, Kalanchoe grandiflora, Kalanchoe hildebrantii, Kalanchoe jongmansii*, *Kalanchoe kewensis*, *Kalanchoe laciniata*, *Kalanchoe laetivirens*, *Kalanchoe lateritia, Kalanchoe laxiflora, Kalanchoe linearifolia, Kalanchoe longiflora, Kalanchoe luciae*, *Kalanchoe macrochlamys*, *Kalanchoe manginii*, *Kalanchoe marnieriana*, *Kalanchoe marmorata*, *Kalanchoe millottii*, *Kalanchoe miniata*, *Kalanchoe nyikae, Kalanchoe obtusa, Kalanchoe orgyalis*, *Kalanchoe peltata*, *Kalanchoe petitiana*, *Kalanchoe pinnata*, *Kalanchoe porphyrocalyx, Kalanchoe prolifera, Kalanchoe pubescens, Kalanchoe pumila, Kalanchoe quartiniana, Kalanchoe rhombopilosa, Kalanchoe robusta, Kalanchoe rolandi, Kalanchoe rosei, Kalanchoe rotundifolia, Kalanchoe schizophylla, Kalanchoe serrata, Kalanchoe sexangularis, Kalanchoe streptantha*, *Kalanchoe suarezensis*, *Kalanchoe synsepala*, *Kalanchoe synsepala f. dissecta*, *Kalanchoe thyrsiflora*, *Kalanchoe tomentosa*, *Kalanchoe tubiflora*, *Kalanchoe uniflora, Kalanchoe velutina* and *Kalanchoe viguieri.*

Most *Kalanchoe* plants are shrubs or perennial herbaceous plants, but a few are annual or biennial. The largest plant, *Kalanchoe beharensis* from Madagascar can reach 6 meters but most species are less than 1 meter. *Kalanchoe* plants are characterized by opening their flowers through growing new cells on the inner surface of the petals to force them outwards and on the outside of the petals to close them.

The *Kalanchoe* genus was first described by the botanist Michel Adanson in 1763. Reportedly, the name came "from the Chinese name for one of the species." This Chinese species is thought to have been either *Kalanchoe ceratophylla* or *Kalanchoe spathulata.* The genus *Bryophyllum* was described by Salisbury in 1806 and the genus *Kitchingia* was created by Baker in 1881. *Kitchingia* is now regarded as a synonym for *Kalanchoe* whereas some botanists treat *Bryophyllum* as a separate genus.

*Kalanchoe* plants are generally cultivated as ornamental houseplants and rock or succulent garden plants. *Kalanchoe* plans are popular because of their ease of propagation, low water requirements, and wide variety of flower colors typically borne in clusters well above the vegetative growth. The section Bryophyllum - formerly an independent genus - contains species such as the "Air plant" *Kalanchoe pinnata.* In these plants, new individuals develop vegetatively as plantlets, also known as bulbils or gemmae, at indents along the leaves. These young plants eventually drop off and root. No males have been found in species of this genus which does flower and produce seeds and is commonly designated as the Mother of Thousands.

In common with other *Crassulaceae* (such as the genera *Tylecodon, Cotyledon* and *Adromischus*), some *Kalanchoe* species contain bufadienolide cardiac glycosides which can cause cardiac poisoning, particularly in grazing animals. This is a particular problem in the native range of many *Kalanchoe* species in the Karoo region of South Africa, where the resulting animal disease is known as krimpsiekte (shrinking disease) or cotyledonosis. Similar poisonings have also occurred in Australia.

In traditional medicine, *Kalanchoe* species have been used to treat ailments such as infections, rheumatism and inflammation. *Kalanchoe* extracts also have immunosuppressive effects. *Kalanchoe pinnata* has been recorded in Trinidad and Tobago as being used as a traditional treatment for hypertension.

A variety of bufadienolide compounds have been isolated from various *Kalanchoe* species. Five different bufadienolides have been isolated from *Kalanchoe daigremontiana.* Two of these, daigremontianin and bersaldegenin 1,3,5-orthoacetate have been shown to have a pronounced sedative effect. They also have the strong positive inotropic effect associated with cardiac glycosides, and with greater doses an increasing effect on the central nervous system. Bufadienolide compounds isolated from *Kalanchoe pinnata* include bryophillin A which showed strong anti-tumor promoting activity and bersaldegenin-3-acetate and bryophillin C which were less active. Bryophillin C also showed insecticidal properties.

As indicated, a number of *Kalanchoe* species, such as *K*. *blossfeldiana, K. laciniata, K. rotundifolia, K. aromatica, K. pubescens, K. grandiflora, K. citrina, K. ambolensis, K. faustii*, *K. schumacherii*, *K. pritwitzii*, *K. flammea*, *K. figueredoi*, *K. rauhii*, *K. obtusa*, *K. pumila, K. marmorata*, *K. porphyrocalux*, *K. jongmansii*, *K. pinnata*, *K. diagremontiana*, *K. gracilipes, K. campanulata, K. latisepela, K. coccinea, K. fedtschenkoi, K. tubiflora, K. decumbens, K. manginii, K. orgyalis, K. crenata, K. tomentosa* and hybrids thereof, are cultivated as ornamental houseplants and rock or succulent garden plants. Accordingly, the appearance, and especially the flowering type of these plants is a major factor determining their economic value and use. One flowering type is the so-called decorative flowering type. *Kalanchoe* plants displaying such decorative flowering type are characterized by having flowers with more than 25 petals, 35 or more than 40, and substantially all flowers of these *Kalanchoe* plants display the decorative flowering phenotype.

Yoshihiro Hase *et al.* (2000) discloses an *Arabidopsis* plant with a mutated FRL1 (Atlg203330) and phenotypes of single and double mutants indicate that the effect of *frl1* is specific to organs with petal or speal identity.

US 18011 P2 (2007) discloses a cultivar of Kalanchoe blossfeldiana having 35-45 petals per flower.

Although *Kalanchoe* plants displaying a decorative flowering phenotype are known in the art, there is a need in the art for genetically causal factors of the decorative flowering phenotype, such as genes, thereby not only facilitating and simplifying the breeding of *Kalanchoe* plants because introgression of the trait can be controlled and selected in relatively early stages of development but also to provide new races of *Kalanchoe* plants, and especially plants having a mixed genetic make-up of two or more *Kalanchoe* species, displaying the decorative flowering phenotype.

Accordingly, it is an objective of the present invention, amongst other objectives, to meet the above need in the art, i.e. providing a genetically causal factor for a decorative flowering phenotype.

This objective, amongst other objectives is met according to the present invention by providing proteins, nucleic acid sequences (cDNA or coding sequences) and genes as outlined in the appended claims.

The present proteins comprise an amino acid substitution of the amino acid histidine at position 136 of the methyltransferase protein of a *Kalanchoe* plant, whereby the present amino acid substitution provides a decorative flowering phenotype in said the present *Kalanchoe* plant and the present proteins comprise an amino acid substitution of the amino acid alanine at position 338 of the methyltransferase protein of a *Kalanchoe* plant, whereby the present amino acid substitution provides a decorative flowering phenotype in the present *Kalanchoe* plant.

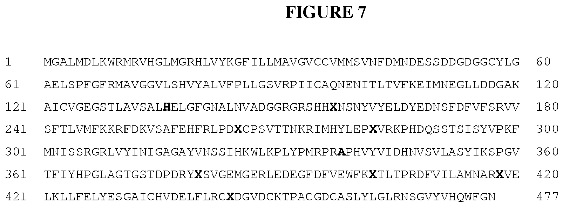

The present proteins comprise the amino acid sequence as depicted in Figure 7 with an amino acid substitution of the amino acid histidine at position 136 and the amino acid alanine at position 338.

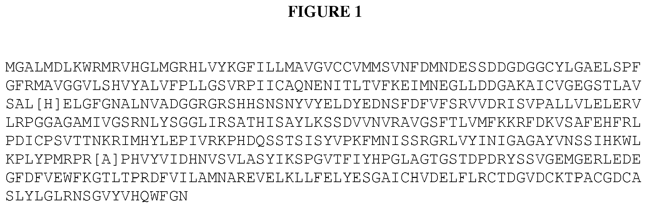

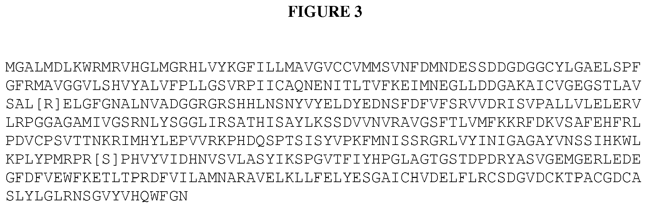

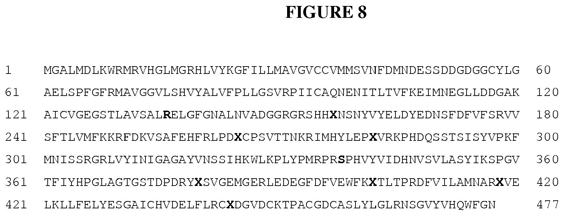

The amino acid sequences shown in Figure 1 and Figure 7 are the amino acid sequences of proteins not providing the decorative flowering phenotype and the amino acid sequences shown in Figure 3 and Figure 8 are the amino acid sequences of a proteins providing the decorative flowering phenotype through a mutation, i.e. in the present case (an) amino acid substitution(s). In Figures 3 and 8, amino acid substitutions, or mutations, are shown but also deletions, truncations and amino acid insertions are contemplated.

The present decorative flowering phenotype can be provided by a single gene mutation, i.e. an amino acid substitution at position 136 and 338 in de coding sequence resulting in a mutated protein. Inheritance experiments have shown that the mutation is dominant, i.e. both for the mutation(s) homozygous and heterozygous *Kalanchoe* plants display the decorative flowering phenotype however heterozygous *Kalanchoe* plants are preferred.

According the present invention, the present proteins comprise an alanine (A or Ala) to serine (S or Ser) amino acid substitution at position 338 and an histidine (H or His) to arginine (R or Arg) amino acid substitution at position 136.

The present proteins comprise the amino acid sequences as shown in Figures 3 or 8. Formulated differently, the present proteins have substantially the amino acid sequences as shown in Figures 3 or 8.

Disclosed are proteins comprising at least one mutation in the amino acid sequence as shown in Figure 1 wherein the at least one mutation provides a decorative flowering phenotype in *Kalanchoe* plants. Having the knowledge that the proteins shown in Figure 1 are a causative factor for the decorative flowering phenotype, the skilled person, using standard means and methods, is readily able to mutate this protein, for example by EMS or site directed mutagenesis followed by an appropriate selection, for example by sequencing or PCR, thereby providing *Kalanchoe* plants showing the decorative flowering phenotype. As an alternative, the present genes can be introduced in a breeding germplasm by conventional breeding.

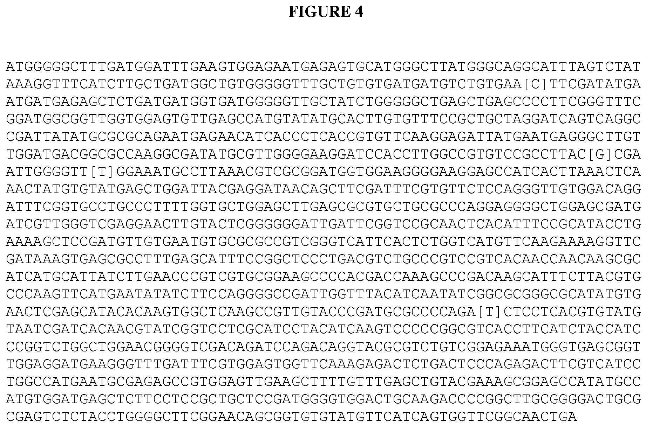

The present invention relates to nucleic acid sequences encoding the present proteins, preferably substantially the nucleic acid sequence as shown in Figure 4.

The present invention relates to genes capable of being transcribed into the present nucleic acid sequences. Formulated differently, the present invention encompasses genes capable of, under appropriate conditions, being a template for transcribing mRNA which mRNA is subsequently translated into the present protein sequences.

The present invention relates to the use of the present proteins, the present nucleic acid sequences or the present genes for selecting decorative flowering *Kalanchoe* plants. Such selection can comprise identification, preferably in an early stage of development, of decorative flowering *Kalanchoe* plants by, for example, hybridization, PCR, ELISA, restriction analysis or Northern/Southern blots thereby facilitating the breeding process of existing and new *Kalanchoe* plants.

The present use is preferably used for the selection of decorative flowering *Kalanchoe* plants selected from the group consisting of *Kalanchoe adelae, Kalanchoe arborescens, Kalanchoe beauverdii, Kalanchoe beharensis, Kalanchoe bentii, Kalanchoe blossfeldiana, Kalanchoe bouvetii, Kalanchoe bracteata, Kalanchoe campanulata, Kalanchoe crenata, Kalanchoe crundallii, Kalanchoe daigremontiana*, *Kalanchoe delagoensis, Kalanchoe dinklagei, Kalanchoe eriophylla, Kalanchoe farinacea, Kalanchoe fedtschenkoi, Kalanchoe figuereidoi, Kalanchoe flammea*, *Kalanchoe gastonis*, *Kalanchoe glaucescens*, *Kalanchoe gracilipes*, *Kalanchoe grandidieri*, *Kalanchoe grandiflora*, *Kalanchoe hildebrantii*, *Kalanchoe jongmansii*, *Kalanchoe kewensis*, *Kalanchoe laciniata*, *Kalanchoe laetivirens*, *Kalanchoe lateritia*, *Kalanchoe laxiflora*, *Kalanchoe linearifolia*, *Kalanchoe longiflora*, *Kalanchoe luciae*, *Kalanchoe macrochlamys*, *Kalanchoe manginii*, *Kalanchoe marnieriana*, *Kalanchoe marmorata*, *Kalanchoe millottii*, *Kalanchoe miniata, Kalanchoe nyikae, Kalanchoe obtusa, Kalanchoe orgyalis, Kalanchoe peltata, Kalanchoe petitiana, Kalanchoe pinnata, Kalanchoe porphyrocalyx, Kalanchoe prolifera, Kalanchoe pubescens, Kalanchoe pumila, Kalanchoe quartiniana, Kalanchoe rhombopilosa, Kalanchoe robusta, Kalanchoe rolandi, Kalanchoe rosei, Kalanchoe rotundifolia, Kalanchoe schizophylla, Kalanchoe serrata, Kalanchoe sexangularis, Kalanchoe streptantha, Kalanchoe suarezensis*, *Kalanchoe synsepala*, *Kalanchoe synsepala f*. *dissecta*, *Kalanchoe thyrsiflora*, *Kalanchoe tomentosa*, *Kalanchoe tubiflora*, *Kalanchoe uniflora*, *Kalanchoe velutina* and *Kalanchoe viguieri*; more preferably decorative flowering *Kalanchoe* plants selected from the group consisting of *K. blossfeldiana*, *K. laciniata*, *K. rotundifolia*, *K. aromatica*, *K. pubescens*, *K. grandiflora*, *K. citrina*, *K. ambolensis*, *K. faustii*, *K. schumacherii*, *K. pritwitzii*, *K. flammea, K. figueredoi*, *K. rauhii*, *K. obtusa*, *K. pumila*, *K. marmorata*, *K. porphyrocalux*, *K. jongmansii*, *K. pinnata*, *K. diagremontiana*, *K. gracilipes*, *K. campanulata*, *K. latisepela*, *K. coccinea*, *K. fedtschenkoi*, *K. tubiflora*, *K. decumbens*, *K. manginii*, *K. orgyalis*, *K. crenata*, *K. tomentosa* and hybrids thereof.

Throughout the description and claims, reference is made to figures wherein:
- **Figure 1:**: shows the amino acid sequence of a protein not causing a decorative flowering phenotype in *Kalanchoe*

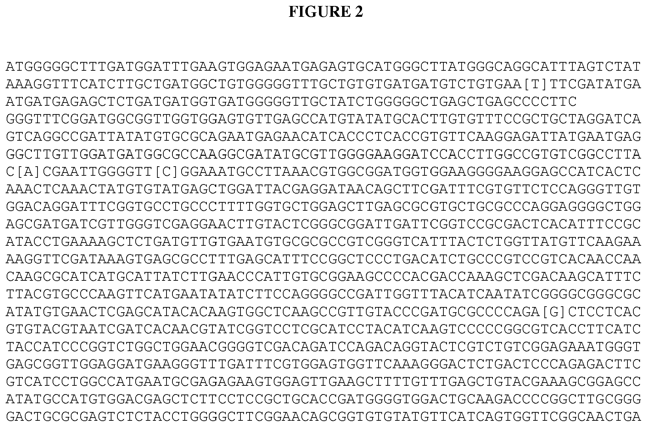

- **Figure 2:**: shows the cDNA sequence encoding the amino acid sequence shown in Figure 1;
- **Figure 3:**: shows the amino acid sequence of a protein causing a decorative flowering phenotype in *Kalanchoe;*
- **Figure 4:**: shows the cDNA sequence encoding the amino acid sequence shown in Figure 3;

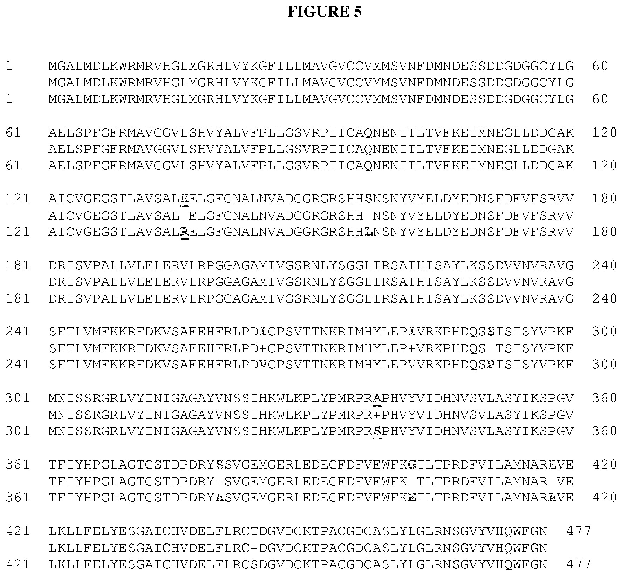

- **Figure 5:**: shows an alignment of the amino acid sequences shown in Figures 1 and 3;

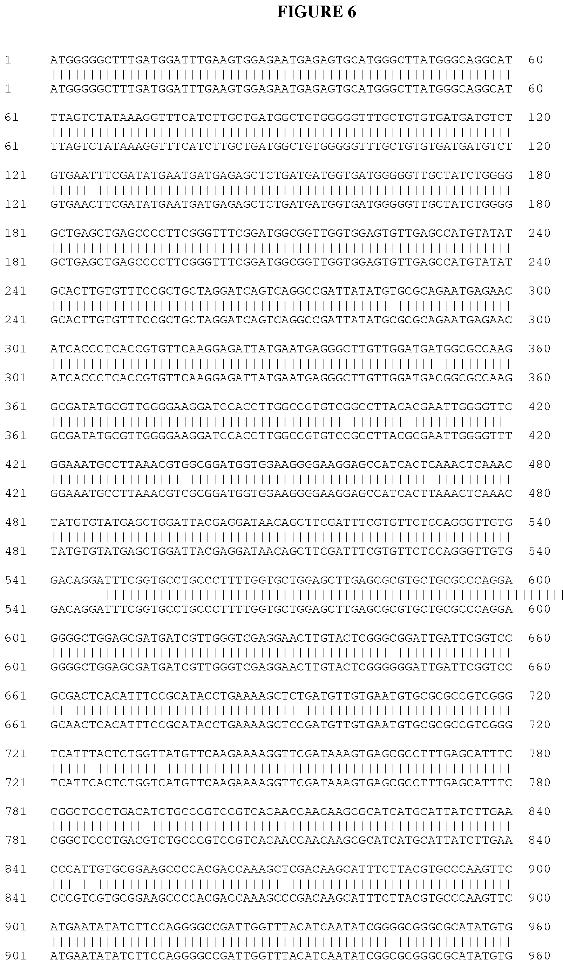

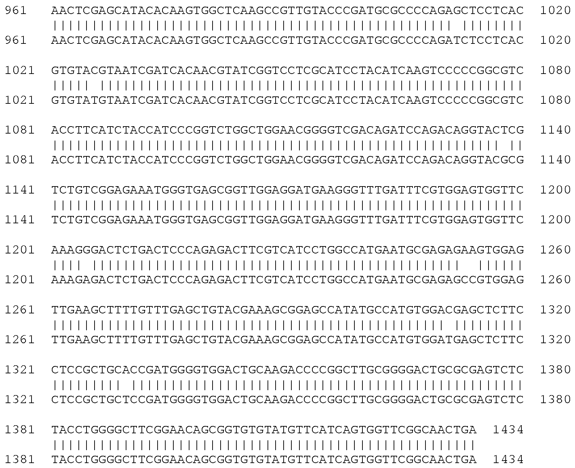

- **Figure 6:**: shows an alignment of the nucleotide sequences shown in Figures 2 and 4;
- **Figure 7:**: shows variable positions denoted with an X (any naturally occurring amino acid) in the amino acid sequence of a protein not causing a decorative flowering phenotype in *Kalanchoe*;
- **Figure 8:**: shows variable positions denoted with an X (any naturally occurring amino acid) in the amino acid sequence of a protein causing a decorative flowering phenotype in *Kalanchoe.*

### Example

The presence of the present methyltransferases as depicted in **Figures 7** **and** **8** was determined in several *Kalanchoe* species having a decorative flowering phenotype. As controls, several non-flowering phenotype *Kalanchoe* species were included. The results are summarized in **Table 1** below.

**Table 1:**

| ***Kalanchoe* variant** | **Decorative flowering phenotype** | **Methyltransferase protein as depicted in** **Figure 7** | **Methyltransferase protein as depicted in** **Figure 8** |
|---|---|---|---|
| Tylo | Yes | Yes | Yes |
| Taylor | Yes | Yes | Yes |
| Jodie | Yes | Yes | Yes |
| African Sunshine | Yes | Yes | Yes |
| Mercedes | Yes | Yes | Yes |
| Don Antonio | Yes | Yes | Yes |
| 12-016-02 | Yes | Yes | Yes |
| Foster | Yes | Yes | Yes |
| Paris | Yes | Yes | Yes |
| Leonardo | Yes | Yes | Yes |
| | | | |
| Alexandra | No | Yes | No |
| Milos | No | Yes | No |
| Snowdon | No | Yes | No |
| Josefine Yellow | No | Yes | No |
| Molly | No | Yes | No |
| African Love | No | Yes | No |
| Amora | No | Yes | No |
| Catalana | No | Yes | No |
| Venetia | No | Yes | No |

As shown in **Table 1**, all decorative flowering *Kalanchoe* species comprised besides a wild-type methyltransferase protein a mutated methyltransferase protein with amino acid substitutions at positions 136 and 338.

## Claims

1. Protein comprising an amino acid substitution of the amino acid histidine at position 136 and the amino acid alanine at position 338 of the methyltransferase protein of a plant, said amino acid substitution provides a decorative flowering phenotype in said plant;
- wherein said protein comprises the amino acid sequence as depicted in figure 8; and
- wherein said decorative flowering phenotype is **characterized by** flowers with at least 25, 35 or more than 40 petals, and substantially all flowers display the decorative flowering phenotype.

2. Protein according to claim 1, wherein said protein comprises the amino acid sequence as shown in Figure 3.

3. Nucleic acid sequence encoding a protein according to claim 1 or claim 2.

4. Nucleic acid sequence as shown in Figure 4.

5. Gene capable of being transcribed into a nucleic acid sequence according to claim 3 or claim 4 or encoding a protein according to claim 1 or claim 2.

6. Use of a protein according to claim 1 or claim 2 or a nucleic acid sequence according to claim 3 or claim 4 or a gene according to claim 5 for selecting decorative flowering *Kalanchoe* plants.

7. Use according to claim 6, wherein said decorative flowering *Kalanchoe* plants are selected from the group consisting of *Kalanchoe adelae, Kalanchoe arborescens, Kalanchoe beauverdii, Kalanchoe beharensis, Kalanchoe bentii, Kalanchoe blossfeldiana, Kalanchoe bouvetii, Kalanchoe bracteata, Kalanchoe campanulata, Kalanchoe crenata, Kalanchoe crundallii, Kalanchoe daigremontiana*, *Kalanchoe delagoensis, Kalanchoe dinklagei, Kalanchoe eriophylla, Kalanchoe farinacea, Kalanchoe fedtschenkoi*, *Kalanchoe figuereidoi, Kalanchoe flammea, Kalanchoe gastonis, Kalanchoe glaucescens, Kalanchoe gracilipes, Kalanchoe grandidieri, Kalanchoe grandiflora, Kalanchoe hildebrantii*, *Kalanchoe jongmansii*, *Kalanchoe kewensis*, *Kalanchoe laciniata*, *Kalanchoe laetivirens*, *Kalanchoe lateritia*, *Kalanchoe laxiflora*, *Kalanchoe linearifolia*, *Kalanchoe longiflora*, *Kalanchoe luciae, Kalanchoe macrochlamys, Kalanchoe manginii, Kalanchoe marnieriana*, *Kalanchoe marmorata, Kalanchoe millottii*, *Kalanchoe miniata, Kalanchoe nyikae, Kalanchoe obtusa, Kalanchoe orgyalis, Kalanchoe peltata*, *Kalanchoe petitiana, Kalanchoe pinnata, Kalanchoe porphyrocalyx*, *Kalanchoe prolifera*, *Kalanchoe pubescens*, *Kalanchoe pumila*, *Kalanchoe quartiniana*, *Kalanchoe rhombopilosa*, *Kalanchoe robusta*, *Kalanchoe rolandi*, *Kalanchoe rosei, Kalanchoe rotundifolia, Kalanchoe schizophylla, Kalanchoe serrata, Kalanchoe sexangularis, Kalanchoe streptantha, Kalanchoe suarezensis, Kalanchoe synsepala*, *Kalanchoe synsepala f*. *dissecta*, *Kalanchoe thyrsiflora*, *Kalanchoe tomentosa*, *Kalanchoe tubiflora, Kalanchoe uniflora, Kalanchoe velutina* and *Kalanchoe viguieri;* preferably decorative flowering *Kalanchoe* plants selected from the group consisting of *K. blossfeldiana*, *K. laciniata*, *K. rotundifolia*, *K. aromatica*, *K. pubescens*, *K. grandiflora*, *K. citrina*, *K. ambolensis*, *K. faustii*, *K. schumacherii*, *K. pritwitzii*, *K. flammea*, *K. figueredoi*, *K. rauhii*, *K. obtusa*, *K. pumila*, *K. marmorata*, *K. porphyrocalux*, *K. jongmansii*, *K. pinnata*, *K. diagremontiana*, *K. gracilipes*, *K. campanulata*, *K. latisepela*, *K. coccinea*, *K. fedtschenkoi*, *K. tubiflora*, *K. decumbens*, *K. manginii*, *K. orgyalis*, *K. crenata*, *K. tomentosa* and hybrids thereof.

8. Use of a protein according to claim 1 or claim 2 or a nucleic acid sequence according to claim 3 or claim 4 or a gene according to claim 5 for development of a molecular marker indicative of, or associated with, a decorative flowering phenotype.

## Patentansprüche

1. Protein, das eine Aminosäuresubstitution der Aminosäure Histidin an der Position 136 und der Aminosäure Alanin an der Position 338 des Methyltransferase-Proteins einer Pflanze aufweist, wobei die Aminosäuresubstitution einen dekorativen blühenden Phänetyp in der Pflanze vorsieht;
- wobei das Protein die Aminosäuresequenz, wie in Fig. 8 dargestellt, aufweist; und
- wobei der dekorative blühende Phänotyp durch Blumen gekennzeichnet ist, die 25, 35 oder mehr als 40 Blüten aufweisen, und alle Blumen im Wesentlichen den dekorativen blühenden Phänotyp darstellen.

2. Protein nach Anspruch 1, wobei das Protein die Aminosäurensequenz, wie in Fig. 3 dargestellt, aufweist.

3. Nukleinsäuresequenz, die ein Protein nach Anspruch 1 oder Anspruch 2 kodiert.

4. Nukleinsäuresequenz, wie in Fig. 4 dargestellt.

5. Gen, das dazu in der Lage ist, in eine Nukleinsäuresequenz nach Anspruch 3 oder Anspruch 4 transkribiert zu werden oder ein Protein nach Anspruch 1 oder Anspruch 2 zu kodieren.

6. Verwenden eines Proteins nach Anspruch 1 oder Anspruch 2 oder einer Nukleinsäuresequenz nach Anspruch 3 oder Anspruch 4 oder eines Gens nach Anspruch 5, um dekorative blühende *Kalanchoe*-Pflanzen auszuwählen.

7. Verwenden nach Anspruch 6,wobei die dekorativen blühenden *Kalanchoe*-Pflanzen ausgewählt sind aus der Gruppe bestehend aus *Kalanchoe adela*, *Kalanchoe arborescens*, *Kalanchoe beauverdii*, *Kalanchoe beharensis*, *Kalanchoe bentii*, *Kalanchoe blossfeldiana*, *Kalanchoe bouvetii*, *Kalanchoe bracteata*, *Kalanchoe campanulata*, *Kalanchoe crenata*, *Kalanchoe crundallii*, *Kalanchoe daigremontiana*, *Kalanchoe delagoensis*, *Kalanchoe dinklagei, Kalanchoe eriophylla*, *Kalanchoe farinacea*, *Kalanchoe fedtschenkoi, Kalanchoe figuereidoi, Kalanchoe flammea, Kalanchoe gastonis, Kalanchoe glaucescens, Kalanchoe gracilipes, Kalanchoe grandidieri, Kalanchoe grandiflora*, *Kalanchoe hildebrantii*, *Kalanchoe jongmansii*, *Kalanchoe*, *kewensis*, *Kalanchoe laciniata, Kalanchoe laetivirens*, *Kalanchoe lateritia*, *Kalanchoe laxiflora*, *Kalanchoe linearifolia*, *Kalanchoe longiflora*, *Kalanchoe luciae*, *Kalanchoe macrochlamys*, *Kalanchoe manginii*, *Kalanchoe marnieriana*, *Kalanchoe marmorata, Kalanchoe millottii*, *Kalanchoe miniata*, *Kalanchoe nyikae*, *Kalanchoe obtusa*, *Kalanchoe orgyalis*, *Kalanchoe peltata*, *Kalanchoe petitiana*, *Kalanchoe pinnata*, *Kalanchoe porphyrocalyx*, *Kalanchoe prolifera*, *Kalanchoe pubescens*, *Kalanchoe pumila*, *Kalanchoe quartiniana*, *Kalanchoe rhombopilosa*, *Kalanchoe robusta*, *Kalanchoe rolandi*, *Kalanchoe rosei*, *Kalanchoe rotundifolia*, *Kalanchoe schizophylla*, *Kalanchoe serrata*, *Kalanchoe sexangularis*, *Kalanchoe streptantha*, *Kalanchoe suarezensis, Kalanchoe synsepala*, *Kalanchoe synsepala f. dissecta, Kalanchoe thyrsiflora*, *Kalanchoe tomentosa*, *Kalanchoe tubiflora*, *Kalanchoe uniflora*, *Kalanchoe velutina* und *Kalanchoe viguierii*; vorzugsweise dekorativen blühenden *Kalanchoe*-Pflanzen, die ausgewählt sind aus der Gruppe bestehend aus *K. blossfeldiana*, *K. laciniata, K. rotundifolia*, *K. aromatica*, *K. pubescens*, *K. grandiflora*, *K. citrina*, *K. ambolensis*, *K. faustii*, *K. schumacherii*, *K. pritwitzii*, *K. flammea*, *K. figueredoi*, *K. rauhii*, *K. obtusa*, *K. pumila*, *K. marmorata*, *K. porphyrocalux*, *K. jongmansii*, *K. pinnata*, *K. diagremontiana*, *K. gracilipes*, *K. campanulata*, *K. latisepela, K. coccinea*, *K. fedtschenkoi*, *K. tubiflora*, *K. decumbens*, *K. manginii*, *K. orgyalis, K. crenata*, *K. tomentosa* und Hybriden davon.

8. Verwenden eines Proteins nach Anspruch 1 oder Anspruch 2 oder einer Nukleinsäuresequenz nach Anspruch 3 oder Anspruch 4 oder eines Gens nach Anspruch 5, um einen molekularen Maker zu entwickeln, der einen dekorativen blühenden Phänotyp angibt oder damit in Beziehung steht.

## Revendications

1. Protéine comprenant une substitution d'acide aminé de l'acide aminé histidine en position 136 et de l'acide aminé alanine en position 338 de la protéine méthyltransférase d'une plante, ladite substitution d'acide aminé introduisant un phénotype de floraison décorative dans ladite plante ;
- dans laquelle ladite protéine comprend la séquence d'acide aminé telle que représentée sur la figure 8 ; et
- dans laquelle ledit phénotype de floraison décorative est **caractérisé par** des fleurs comportant au moins 25, 35 ou plus de 40 pétales, et sensiblement toutes les fleurs présentent le phénotype de floraison décorative.

2. Protéine selon la revendication 1, dans laquelle ladite protéine comprend la séquence d'acide aminé telle que représentée sur la figure 3.

3. Séquence d'acide nucléique codant pour une protéine selon la revendication 1 ou la revendication 2.

4. Séquence d'acide nucléique telle que représentée sur la figure 4.

5. Gène pouvant être transcrit en une séquence d'acide nucléique selon la revendication 3 ou la revendication 4 ou codant pour une protéine selon la revendication 1 ou la revendication 2.

6. Utilisation d'une protéine selon la revendication 1 ou la revendication 2 ou d'une séquence d'acide nucléique selon la revendication 3 ou la revendication 4 ou d'un gène selon la revendication 5 pour sélectionner des plantes de *Kalanchoe* à floraison décorative.

7. Utilisation selon la revendication 6, dans laquelle lesdites plantes de *Kalanchoe* à floraison décorative sont sélectionnées dans le groupe constitué de *Kalanchoe adelae*, *Kalanchoe arborescens*, *Kalanchoe beauverdii*, *Kalanchoe beharensis*, *Kalanchoe bentii*, *Kalanchoe blossfeldiana, Kalanchoe bouvetii, Kalanchoe bracteata, Kalanchoe campanulata, Kalanchoe crenata, Kalanchoe crundallii*, *Kalanchoe daigremontiana*, *Kalanchoe delagoensis, Kalanchoe dinklagei*, *Kalanchoe eriophylla, Kalanchoe farinacea, Kalanchoe fedtschenkoi*, *Kalanchoe figuereidoi, Kalanchoe flammea*, *Kalanchoe gastonis, Kalanchoe glaucescens*, *Kalanchoe gracilipes, Kalanchoe grandidieri, Kalanchoe grandiflora, Kalanchoe hildebrantii*, *Kalanchoe jongmansii*, *Kalanchoe kewensis*, *Kalanchoe laciniata, Kalanchoe laetivirens, Kalanchoe lateritia, Kalanchoe laxiflora, Kalanchoe linearifolia, Kalanchoe longiflora, Kalanchoe luciae*, *Kalanchoe macrochlamys, Kalanchoe manginii, Kalanchoe marnieriana*, *Kalanchoe marmorata, Kalanchoe millottii*, *Kalanchoe miniata, Kalanchoe nyikae, Kalanchoe obtusa, Kalanchoe orgyalis*, *Kalanchoe peltata, Kalanchoe petitiana, Kalanchoe pinnata, Kalanchoe porphyrocalyx*, *Kalanchoe prolifera*, *Kalanchoe pubescens*, *Kalanchoe pumila*, *Kalanchoe quartiniana*, *Kalanchoe rhombopilosa*, *Kalanchoe robusta*, *Kalanchoe rolandi*, *Kalanchoe rosei*, *Kalanchoe rotundifolia*, *Kalanchoe schizophylla, Kalanchoe serrata*, *Kalanchoe sexangularis*, *Kalanchoe streptantha*, *Kalanchoe suarezensis*, *Kalanchoe synsepala*, *Kalanchoe synsepala f dissecta*, *Kalanchoe thyrsiflora, Kalanchoe tomentosa*, *Kalanchoe tubiflora, Kalanchoe uniflora, Kalanchoe velutina* et *Kalanchoe viguieri* ; de préférence des plantes de *Kalanchoe* à floraison décorative sélectionnées dans le groupe constitué de *K. blossfeldiana*, *K. laciniata, K. rotundifolia*, *K. aromatica*, *K. pubescens*, *K. grandiflora*, *K. citrina*, *K. ambolensis*, *K. faustii*, *K. schumacherii*, *K. pritwitzii*, *K. flammea*, *K. figueredoi*, *K. rauhii*, *K. obtusa*, *K. pumila*, *K. marmorata*, *K. porphyrocalux*, *K. jongmansii*, *K. pinnata*, *K. diagremontiana*, *K. gracilipes*, *K. campanulata*, *K. latisepela*, *K. coccinea*, *K. fedtschenkoi*, *K. tubiflora*, *K. decumbens*, *K. manginii*, *K. orgyalis*, *K. crenata*, *K. tomentosa* et des hybrides de celles-ci.

8. Utilisation d'une protéine selon la revendication 1 ou la revendication 2 ou d'une séquence d'acide nucléique selon la revendication 3 ou la revendication 4 ou d'un gène selon la revendication 5 pour le développement d'un marqueur moléculaire indicatif de ou associé à un phénotype de floraison décorative.
